# EUROPÄISCHE PATENTSCHRIFT

(11) **EP 0 778 832 B1**
(45) Veröffentlichungstag und Bekanntmachung des Hinweises auf die Patenterteilung: **11.11.1998**
(21) Anmeldenummer: 95930486.6
(22) Anmeldetag: 17.08.1995
(51) Int. Cl.: C07D 295/14, C07C 255/24

(54) **VERFAHREN ZUR HERSTELLUNG VON QUATERNIERTEN GLYCINNITRILEN**
PROCESS FOR PRODUCING QUATERNARY GLYCINE NITRILES
PROCEDE DE PREPARATION DE NITRILES DE GLYCINE QUATERNAIRES

(30) Priorität: 02.09.1994 DE 4431212
(43) Veröffentlichungstag der Anmeldung: 18.06.1997
(73) Patentinhaber: BASF AKTIENGESELLSCHAFT, 67056 Ludwigshafen (DE)
(72) Erfinder: ANNEN, Ulrich, D-67454 Hassloch (DE); SEELMANN-EGGEBERT, Hans-Peter, D-67117 Limburgerhof (DE); WIDDER, Rudi, D-69181 Leimen (DE); MÜLLER, Reinhard, D-67159 Friedelsheim (DE)
(86) Internationale Anmeldenummer: EP9503273
(87) Internationale Veröffentlichungsnummer: WO9607650

(56) Entgegenhaltungen:
- EP-A- 0 049 577
- EP-A- 0 458 396
- EP-A- 0 464 880
- JOURNAL OF THE AMERICAN CHEMICAL SOCIETY, Bd.56, 1934, WASHINGTON, DC US Seiten 2151 - 2158 D.B. LUTEN; T.D. STEWART 'The kinetics of the alkaline hydrolysis of the betaine amides'

## Beschreibung

Die vorliegende Erfindung betrifft ein verbessertes Verfahren zur Herstellung von quaternierten Glycinnitrilen der allgemeinen Formel I 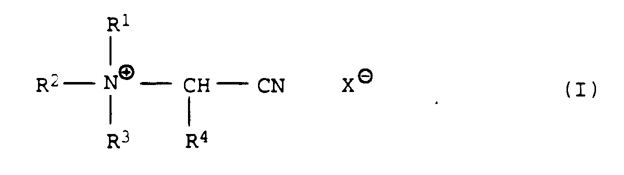 in der
- R¹ und R²: gleich oder verschieden sein können und jeweils einen aliphatischen, cycloaliphatischen oder araliphatischen Rest mit 1 bis 30 C-Atomen bedeuten, wobei R¹ und R² auch zusammen mit dem N-Atom, an das sie gebunden sind, einen gesättigten oder ungesättigten fünf- oder sechsgliedrigen heterocyclischen Ring bilden können, welcher zusätzlich weitere Heteroatome enthalten, benzanelliert sein und Alkylseitengruppen tragen kann, und darüber hinaus R² auch Wasserstoff bedeuten kann,
- R³: C₁- bis C₄-Alkyl oder Benzyl bezeichnet,
- R⁴: für Wasserstoff, C₁- bis C₂₀-Alkyl, welches durch ein oder mehrere nicht benachbarte Sauerstoffatome unterbrochen sein kann, oder einen Rest der Formel 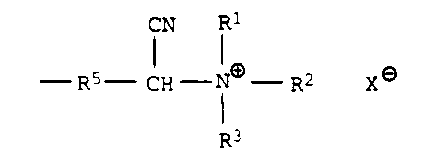 in der R⁵ eine chemische Bindung oder eine C₁- bis C₆-Alkylenbrücke bedeutet, steht, und
- X^{⊖}: ein Gegenion bezeichnet,
durch Umsetzung von Aminen der allgemeinen Formel II 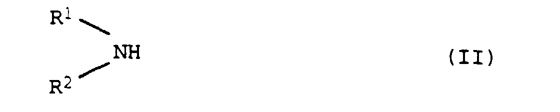 in der die Variablen R¹ und R² die oben genannte Bedeutung haben, mit einem Monoaldehyd der allgemeinen Formel IIIa oder einem Dialdehyd der allgemeinen Formel IIIb

R⁶ ― CHO (IIIa)

OHC ― R⁵― CHO (IIIb)

wobei R⁵ die oben genannte Bedeutung hat und R⁶ für Wasserstoff oder C₁- bis C₂₀-Alkyl steht, und Blausäure oder einem Alkalimetallcyanid in wäßrigem Medium und anschließender Quaternierung mit einem Alkylierungsmittel der allgemeinen Formel IV

R³―X (IV)

in der R³ die oben genannte Bedeutung hat und X eine Abgangsgruppe darstellt.

Aus den Schriften US-A 5 236 616 (1) und US-A 5 281 361 (2) sind kationische Nitrile mit beispielsweise der Struktureinheit 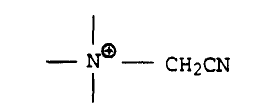 bekannt. Kationische Nitrile, z.B. (CH₃)₃N^{⊕}-CH₂CN Cl^{⊖}, werden dort als äußerst hygroskopische, d.h. wasserempfindliche Substanzen dargestellt, welche rasch aus der Luft Wasser aufnehmen und leicht zu entsprechenden Amiden hydrolysieren können. Kationische Nitrile werden deshalb in aller Regel in wasserfreien organischen Lösungsmitteln, z.B. in trockenem Acetonitril (vgl. Beispiel 1(i) von (1)), hergestellt.

Der vorliegenden Erfindung lag daher die Aufgabe zugrunde, ein Herstellungsverfahren für kationische Nitrile oder Formulierungen hieraus bereitzustellen, welches Produkte liefert, die eine solche Wasserempfindlichkeit nicht zeigen.

Demgemäß wurde das eingangs definierte Verfahren gefunden, welches dadurch gekennzeichnet ist, daß man die Umsetzung der Amine II mit den Aldehyden III und Blausäure bzw. Alkalimetallcyanid und die Quaternierung nacheinander ohne Isolierung eines Zwischenproduktes in wäßrigem Medium vornimmt.

Man erhält hierbei wäßrige Lösungen der beschriebenen quaternierten Glycinnitrile I, die unter üblichen Bedingungen praktisch unbegrenzt stabil, d.h. nicht hydrolyseempfindlich sind. Üblicherweise werden bei Lagerung der erfindungsgemäß hergestellten wäßrigen Lösungen der quaternierten Glycinnitrile I selbst nach einem Zeitraum von mehreren Wochen bei Temperaturen von bis zu 40°C keinerlei Hydrolyseprodukte gefunden.

Die Vorstufen der nicht quaternierten Glycinnitrile I, welche beim erfindungsgemäßen Verfahren ja nicht isoliert oder gar gereinigt werden, und ihre Herstellung sind im Prinzip aus den Schriften DE-A 25 03 582 (3), DE-A 25 55 769 (4) und DE-A 26 20 445 (5) bekannt.

Die Reste R¹ und R² bedeuten jeweils insbesondere C₁- bis C₁₈-Alkylgruppen, vor allem C₂- bis C₁₀-Alkylgruppen, weiterhin insbesondere C₃- bis C₁₀-Cycloalkylgruppen, vor allem C₅- bis C₇-Cycloalkylgruppen, sowie weiterhin insbesondere araliphatische Reste mit 7 bis 20 C-Atomen, vor allem Phenylalkylgruppen mit 7 bis 12 C-Atomen.

Als heterocyclische Ringstrukturen, die R¹ und R² enthalten, kommen insbesondere solche in Betracht, die neben dem aus dem Glycinteil stammenden N-Atom kein, ein oder zwei weitere Heteroatome aus der Gruppe Stickstoff oder Sauerstoff enthalten. Die heterocyclischen Ringe tragen vorzugsweise keinen, einen oder zwei Benzanellanten. Treten zusätzliche Alkylseitengruppen auf, handelt es sich hierbei vorzugsweise um C₁- bis C₄-Alkylgruppen, insbesondere Methyl oder Ethyl.

Entsprechend den aufgezählten Bedeutungen für R¹ und R² können beim erfindungsgemäßen Verfahren die folgenden primären oder sekundären Amine II als Ausgangsverbindungen mit besonders guten Resultaten eingesetzt werden:
Methyl-, Ethyl-, n-Propyl-, Isopropyl-, n-Butyl-, Isobutyl-, sek.-Butyl-, tert.-Butyl-, Pentyl-, Pentyl-(2)-, Pentyl-(3)-, n-Hexyl-, n-Heptyl-, n-Octyl-, n-Nonyl, n-Decyl-, 2,2,6-Trimethyl-n-pentyl-, 2-Ethylpentyl-, 3-Ethylpentyl-, 2,3-Dimethyl-n-butyl-, 2,2-Dimethyl-n-butyl-, 2-Methylpentyl-, 3-Methylpentyl-, 2,2,4-Trimethylpentyl-, 2-Methylheptyl-, 3-Methylheptyl, 4-Methylheptyl, 2-Ethylhexyl-, 3-Ethylhexyl-, 2,2-Dimethylhexyl-, 2,3-Dimethylhexyl-, 2,4-Dimethylhexyl-, 2,5-Dimethylhexyl-, 3,3-Dimethylhexyl-, 3,4-Dimethylhexyl-, 2-Methyl-3-ethylpentyl-, 3-Methyl-3-ethylpentyl-, 2,2,3-Trimethylpentyl-, 2,2,4-Trimethylpentyl-, 2,3,3-Trimethylpentyl-, 2,3,4-Trimethylpentyl-, 2,2,3,3-Tetramethylbutyl-amin;
Di-(methyl)-, Di-(ethyl)-, Di-(n-propyl)-, Di-(isopropyl)-, Di-(n-butyl)-, Di-(isobutyl)-, Di-(sek.-butyl)-, Di-(tert.-butyl)-, Di-(pentyl)-, Di-(pentyl)-(2)-, Di-(pentyl)-(3)-, Di-(n-hexyl)-, Di-(n-heptyl)-, Di-(n-octyl)-, Di-(n-nonyl)-, Di-(n-decyl)-, Di-(2,2,6-trimethyl-n-pentyl)-, Di-(2-ethylpentyl)-, Di-(3-ethylpentyl)-, Di-(2,3-dimethyl-n-butyl)-, Di-(2,2-dimethyl-n-butyl)-, Di-(2-methylpentyl)-, Di-(3-methylpentyl)-, Di-(2,2,4-trimethylpentyl)-, Di-(2-methylheptyl)-, Di-(3-methylheptyl)-, Di-(4-methylheptyl)-, Di-(2-ethylhexyl)-, Di-(3-ethylhexyl)-, Di-(2,2-dimethylhexyl)-, Di-(2,3-dimethylhexyl)-, Di- (2,4-dimethylhexyl)-, Di-(2,5-dimethylhexyl)-, di-(3,3-dimethylhexyl)-, Di-(3,4-dimethylhexyl)-, Di- (2-methyl-3-ethylpentyl)-, Di-(3-methyl-3-ethylpentyl)-, Di-(2,2,3-trimethylpentyl)-, Di-(2,2,4-trimethylpentyl)-, Di-(2,3,3-trimethylpentyl)-, Di-(2,3,4-trimethylpentyl)-, di-(2,2,3,3-tetramethylbutyl)-amin;
entsprechende aliphatische Amine mit zwei der vorgenannten, aber unterschiedlichen Resten, z.B. Methylethylamin;
Cyclohexylamin, Cyclopentylamin, Cycloheptylamin, Benzylamin, Phenylethylamin;
Dicyclohexylamin, Dicyclopentylamin, Dicycloheptylamin, Dibenzylamin, Diphenylethylamin;
Pyrrolidin, Δ²-Pyrrolin, Δ³-Pyrrolin, Pyrrol, Pyrazol, Pyrazolin, Pyrazolidin, Imidazolidin, Hexamethylenimin, 3-Imidazolin, Piperidin, Piperazin, Indolin, Indol, Isoindolin, Isoindol, Indazol, Benzimidazol, 1,2,3,4-Tetrahydroisochinolin, Carbazol, Phenoxazin, 4-Methylimidazol, 2-Methylindol, 3-Methylindol, 2-Methylpiperazin, 3-Methylpyrrol, 2-Methylpyrrol, 2-Ethylpiperidin, 2-Methylpyrrolidin.

Als Alkylierungsmittel IV, welches für die Einführung der Gruppe R³ verantwortlich ist, kommt insbesondere Dimethylsulfat, Diethylsulfat, ein Methyl- oder Ethylhalogenid, Dimethylcarbonat, Diethylcarbonat, Methyltosylat, Ethyltosylat, Methylmesylat, Ethylmesylat oder ein Benzylhalogenid in Betracht. Unter Halogeniden sind hier Chlorid, Bromid oder Iodid zu verstehen. Dementsprechend sind auch die bevorzugten Bedeutungen für die Abgangsgruppe X bzw. das Gegenion X^{⊖}OCH₃OSO₃, C₂H₅OSO₃, Cl, Br, I, CH₃OCO₂, C₂H₅OCO₂, p-Tolyl-SO₃ und CH₃SO₃. Bevorzugte Bedeutungen für R³ sind demgemäß Methyl, Ethyl und Benzyl.

Der Rest R⁴, welcher aus der Aldehyd-Komponente III stammt, steht vorzugsweise für Wasserstoff, was der Ausgangsverbindung Formaldehyd als Verbindung IIIa entspricht. Es können aber auch C₂- bis C₂₁-Alkanale, insbesondere C₂- bis C₇-Alkanale als Verbindungen IIIa eingesetzt werden, z.B. Acetaldehyd, Propionaldehyd oder Butyraldehyd. Werden Dialdehyde IIIb wie Glyoxal, Malondialdehyd, Succindialdehyd, Glutardialdehyd, 3-Oxaglutardialdehyd oder Adipindialdehyd verwendet, tritt bei entsprechenden stöchiometrischen Verhältnissen in der Regel eine Verdoppelung der Glycinnitril-Struktur auf.

Die Umsetzung der Amine II mit den Aldehyden III und Blausäure bzw. Alkalimetallcyanid zu den nicht quaternierten Glycinnitrilen I in wäßrigem Medium ist im Prinzip aus den Schriften (3) bis (5) bekannt. Normalerweise arbeitet man bei Temperaturen von 0 bis 80°C, insbesondere 20 bis 65°C, vor allem 30 bis 55°C, und bei Normaldruck. Die Umsetzung ist meist nach 4 bis 6 Stunden beendet.

Die sich anschließende Quaternierung mittels des Alkylierungsmittels IV wird in der Regel im gleichen Temperatur- und Druckbereich durchgeführt.

Die erfindungsgemäß als "Eintopfreaktion" ausgestaltete Herstellung der Produkte I durch Umsetzung der Amine II und durch Quaternierung wird vorteilhafterweise entweder in rein wäßriger Lösung oder einer Mischung aus Wasser und bis zu 30 Gew.-%, insbesondere bis zu 15 Gew.-%, vor allem bis zu 5 Gew.-%, bezogen auf die Mischung, eines wassermischbaren organischen Lösungsmittels, beispielsweise eines Alkohols wie Methanol, Ethanol oder Isopropanol, vorgenommen.

### Beispiel

### Herstellung von N-Methylpiperidiniumacetonitril-methosulfat

In einem Rührgefäß legte man 341 g (4 mol) Piperidin vor. Bei 40°C wurden gleichzeitig 400 g (4 mol) einer 30 gew.-%igen wäßrigen Formaldehyd-Lösung und 108 g (4 mol) Blausäure innerhalb von 2 Stunden zudosiert. Nach 1 Stunde Nachrühren bei 40°C gab man bei derselben Temperatur 504 g (4 mol) Dimethylsulfat zügig zu und ließ 2 Stunden bei 40°C nachrühren. In der erhaltenen Lösung war kein Dimethylsulfat mehr nachweisbar.

## Patentansprüche

1. Verfahren zur Herstellung von quaternierten Glycinnitrilen der allgemeinen Formel I 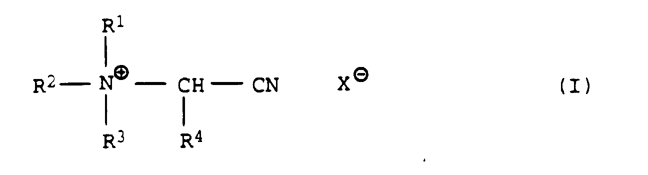 in der
R¹ und R² gleich oder verschieden sein können und jeweils einen aliphatischen, cycloaliphatischen oder araliphatischen Rest mit 1 bis 30 C-Atomen bedeuten, wobei R¹ und R² auch zusammen mit dem N-Atom, an das sie gebunden sind, einen gesättigten oder ungesättigten fünf- oder sechsgliedrigen heterocyclischen Ring bilden können, welcher zusätzlich weitere Heteroatome enthalten, benzanelliert sein und Alkylseitengruppen tragen kann, und darüber hinaus R² auch Wasserstoff bedeuten kann,
R³ C₁- bis C₄-Alkyl oder Benzyl bezeichnet,
R⁴ für Wasserstoff, C₁- bis C₂₀-Alkyl, welches durch ein oder mehrere nicht benachbarte Sauerstoffatome unterbrochen sein kann, oder einen Rest der Formel 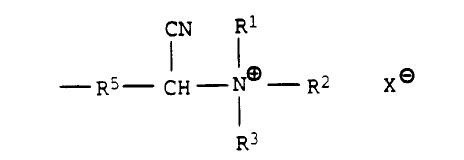 in der R⁵ eine chemische Bindung oder eine C₁- bis C₆-Alkylenbrücke bedeutet, steht, und
X^{⊖} ein Gegenion bezeichnet,
durch Umsetzung von Aminen der allgemeinen Formel II 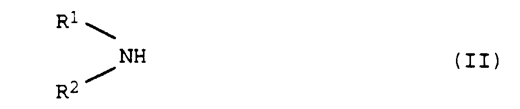 in der die Variablen R¹ und R² die oben genannte Bedeutung haben, mit einem Monoaldehyd der allgemeinen Formel IIIa oder einem Dialdehyd der allgemeinen Formel IIIb
R⁶―CHO (IIIa)
OHC―R⁵―CHO (IIIb)
wobei R⁵ die oben genannte Bedeutung hat und R⁶ für Wasserstoff oder C₁- bis C₂₀-Alkyl steht, und Blausäure oder einem Alkalimetallcyanid in wäßrigem Medium und anschließender Quaternierung mit einem Alkylierungsmittel der allgemeinen Formel IV
R³―X (IV)
in der R³ die oben genannte Bedeutung hat und X eine Abgangsgruppe darstellt, dadurch gekennzeichnet, daß man die Umsetzung der Amine II mit den Aldehyden III und Blausäure bzw. Alkalimetallcyanid und die Quaternierung nacheinander ohne Isolierung eines Zwischenproduktes in wäßrigem Medium vornimmt.

2. Verfahren zur Herstellung von quaternierten Glycinnitrilen I nach Anspruch 1, bei denen R⁴ für Wasserstoff steht.

3. Verfahren zur Herstellung von quaternierten Glycinnitrilen I nach Anspruch 1 oder 2, wobei als Alkylierungsmittel IV Dimethylsulfat, Diethylsulfat, ein Methyl- oder Ethylhalogenid, Dimethylcarbonat, Diethylcarbonat, Methyltosylat, Ethyltosylat, Methylmesylat, Ethylmesylat oder ein Benzylhalogenid eingesetzt wird.

4. Verfahren zur Herstellung von quaternierten Glycinnitrilen I nach den Ansprüchen 1 bis 3, dadurch gekennzeichnet, daß man die Umsetzung der Amine II und die Quaternierung in rein wäßriger Lösung oder einer Mischung aus Wasser und bis zu 30 Gew.-%, bezogen auf die Mischung, eines wassermischbaren organischen Lösungsmittels vornimmt.

## Claims

1. A process for preparing quaternized glycine nitriles of the general formula I 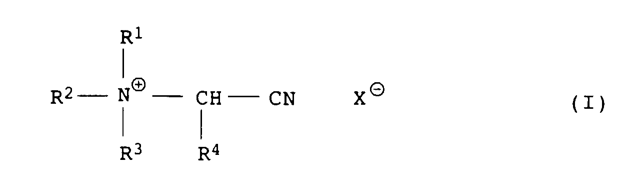 where
R¹ and R² can be identical or different and are each an aliphatic, cycloaliphatic or araliphatic radical having from 1 to 30 carbon atoms, with R¹ and R² also being able to form, together with the nitrogen atom to which they are bound, a saturated or unsaturated five-membered or six-membered heterocyclic ring which can additionally contain further hetero atoms, can be benzo-fused and can bear alkyl side groups, and furthermore R² can also be hydrogen,
R³ is C₁- to C₄-alkyl or benzyl,
R⁴ is hydrogen, C₁- to C₂₀-alkyl which can be interrupted by one or more non-adjacent oxygen atoms, or a radical of the formula 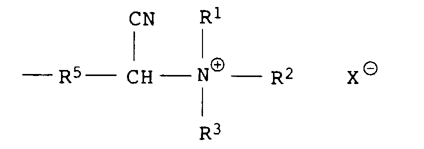 where R⁵ is a chemical bond or a C₁- to C₆-alkylene bridge, and
X^{⊖} is a counter ion,
by reaction of amines of the general formula II 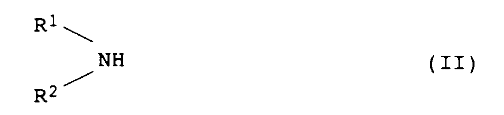 where the variables R¹ and R² are as defined above, with a monoaldehyde of the general formula IIIa or a dialdehyde of the general formula IIIb
R⁶―CHO (IIIa)
OHC―R⁵―CHO (IIIb)
where R⁵ is as defined above and R⁶ is hydrogen or C₁- to C₂₀-alkyl, and hydrocyanic acid or an alkali metal cyanide in aqueous medium and subsequent quaternization with an alkylating agent of the general formula IV
R³―X (IV)
where R³ is as defined above and X is a leaving group, wherein the reaction of the amines II with the aldehydes III and hydrocyanic acid or alkali metal cyanide and the quaternization are carried out successively in aqueous medium without isolation of an intermediate.

2. A process for preparing quaternized glycine nitriles I as claimed in claim 1, in which R⁴ is hydrogen.

3. A process for preparing quaternized glycine nitriles I as claimed in claim 1 or 2, wherein the alkylating agent IV used is dimethyl sulfate, diethyl sulfate, a methyl or ethyl halide, dimethyl carbonate, diethyl carbonate, methyl tosylate, ethyl tosylate, methyl mesylate, ethyl mesylate or a benzyl halide.

4. A process for preparing quaternized glycine nitriles I as claimed in any of claims 1 to 3, wherein the reaction of the amines II and the quaternization are carried out in pure aqueous solution or a mixture of water and up to 30% by weight, based on the mixture, of a water-miscible organic solvent.

## Revendications

1. Procédé de préparation de nitriles de glycine quaternaires de formule générale I 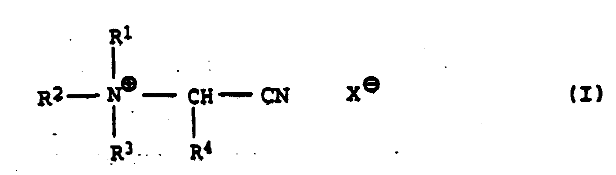 dans laquelle
R¹ et R² sont identiques ou différents et représentent chacun un reste aliphatique, cycloaliphatique ou araliphatique ayant de 1 à 30 atomes de carbone, où R¹ et R² peuvent aussi représenter ensemble, avec l'atome d'azote auxquels ils sont liés, un cycle hétérocyclique saturé ou insaturé à cinq ou six maillons, pouvant en outre contenir d'autres hétéréoatomes, être benzocondensé et porter des groupements alkyl latéraux, R² pouvant en outre représenter un atome d'hydrogène,
R³ représente un groupement alkyle en C₁-C₄ ou benzyle,
R⁴ représente un atome d'hydrogène, un groupement alkyle en C₁-C₂₀, pouvant être interrompu par un ou plusieurs atomes d'oxygène non voisins, ou un reste de formule 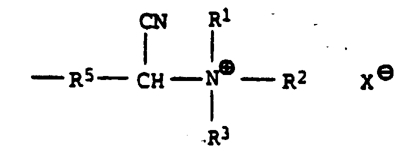 dans laquelle R⁵ représente une liaison chimique ou un pont alkylène en C₁-C₆, et
X^{⊖} représente un contre-ion,
par réaction d'amines de formule générale II 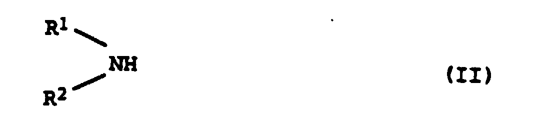 dans laquelle les variables R¹ et R² prennent la signification susmentionnée, avec un monoaldéhyde de formule générale IIIa ou un dialdéhyde de formule générale IIIb
R⁶―CHO (IIIa)
OHC―R⁵―CHO (IIIb)
où R⁵ prend la signification susmentionnée et R⁶ est mis pour un atome d'hydrogène ou un groupement alkyle en C₁-C₂₀, et de l'acide cyanhydrique ou un cyanure de métal alcalin en milieu aqueux, suivie d'une quaternisation avec un agent d'alkylation de formule générale Iv
R³―X (IV)
dans laquelle R³ prend la signification susmentionnée et X représente un groupe partant, caractérisé en ce que l'on mène la réaction de l'amine II avec les aldéhydes III et l'acide cyanhydrique, respectivement le cyanure de métal alcalin et la quaternisation l'une à la suite de l'autre sans isoler un produit intermédiaire en milieu aqueux.

2. Procédé de préparation de nitriles de glycine I quaternaires selon la revendication 1, dans lequel R⁴ est mis pour un atome d'hydrogène.

3. Procédé de préparation de nitriles de glycine I quaternaires selon la revendication 1 ou 2, où on utilise en tant qu'agent d'alkylation IV, du sulfate de diméthyle, du sulfate de diéthyle, un halogénure de méthyle ou d'éthyle, du carbonate de diméthyle, du carbonate de diéthyle, du tosylate de méthyle, du tosylate d'éthyle, du mésylate de méthyle, du mésylate d'éthyle ou un halogénure de benzyle.

4. Procédé de préparation de nitriles de glycine I quaternaires selon l'une quelconque des revendications 1 à 3, caractérisé en ce que l'on mène la réaction de l'amine II et la quaternisation dans une solution aqueuse pure ou dans un mélange d'eau et de jusqu'à 30% en poids, par rapport au mélange, d'un solvant organique miscible à l'eau.
